# EUROPÄISCHE PATENTSCHRIFT

(11) **EP 2 478 055 B1**
(45) Veröffentlichungstag und Bekanntmachung des Hinweises auf die Patenterteilung: **17.06.2015**
(21) Anmeldenummer: 10747226.8
(22) Anmeldetag: 31.08.2010
(51) Int. Cl.: C08L 83/04, A61K 8/892, A61K 8/893, A61Q 5/02, A61Q 5/12, C08J 3/03, C08J 3/22, C08K 5/521

(54) **SILICONEMULSIONEN UND VERFAHREN ZU DEREN HERSTELLUNG**
SILICON EMULSIONS AND PROCESS FOR THE PRODUCTION THEREOF
ÉMULSIONS DE SILICONE ET PROCÉDÉ DE PRODUCTION

(30) Priorität: 16.09.2009 DE 102009029520
(43) Veröffentlichungstag der Anmeldung: 25.07.2012
(73) Patentinhaber: Wacker Chemie AG, 81737 München (DE)
(72) Erfinder: RAUTSCHEK, Holger, 01612 Nünchritz (DE)
(74) Vertreter: Budczinski, Angelika
(86) Internationale Anmeldenummer: PCT/EP2010/062667
(87) Internationale Veröffentlichungsnummer: WO 2011/032824

(56) Entgegenhaltungen:
- DE-A1- 2 014 174
- DE-A1- 2 730 923
- GB-A- 1 246 134
- US-B1- 6 448 297

## Beschreibung

Die Erfindung betrifft wässrige Siliconemulsionen, die hochviskose Polyorganosiloxane enthalten und einen besonders niedrigen Gehalt an cyclischen Siloxanen aufweisen, Verfahren zu deren Herstellung sowie deren Verwendung.

Silicone sind vielfältig einsetzbar. Um die Anwendung und Dosierung insbesondere bei viskosen Produkten zu erleichtern, ist es für viele Anwendungen wünschenswert, dass die siliziumorganischen Verbindungen in verdünnter Form vorliegen. Die Verwendung von organischen Lösemitteln, wie Benzol oder Chlorkohlenwasserstoffen, für diesen Zweck ist zwar möglich, jedoch aus ökologischer und arbeitsmedizinischer Sicht nachteilig. Deshalb erfolgt der Einsatz meist in Form von wässrigen Emulsionen oder Dispersionen, üblicherweise als Öl-in-Wasser-Emulsionen (O/W-Emulsionen), die mit Wasser verdünnbar sind. Als Ölphase werden dabei die mit Wasser nicht mischbaren siliziumorganischen Verbindungen verstanden, gegebenenfalls gelöst in organischen Lösemitteln.

Für viele Anwendungen ist es vorteilhaft, wenn das Silicon ein hohes Molekulargewicht und somit eine hohe Viskosität hat. Ein bekannter Weg zu Emulsionen zu gelangen, die ein hochmolekulares Silicon enthalten, ist die Emulsionspolymerisation von niedermolekularen, insbesondere cyclischen Organosiloxanen mit Arylalkylsulfonsäuren (DE-OS 14 95 512). Dabei werden durch intensives Rühren oder Homogenisieren mit einem Hochdruckhomogenisator außergewöhnlich niedrige Teilchengrößen erreicht, die mit einem optischen Mikroskop nicht mehr erkennbar sind. Nachteilig bei diesem Verfahren ist die Tatsache, dass aufgrund des Gleichgewichtscharakters dieser Reaktion bezogen auf Siloxan über 10% flüchtige cyclische Siloxane enthalten sind, die jedoch unerwünscht sind. Deshalb ist vorgeschlagen worden, diese nachträglich abzudestillieren (z.B. US-A 4,600,436) oder mit einem Membranverfahren zu entfernen (EP-A 1 368 109). Beide Verfahren bedeuten zusätzlichen technischen Aufwand und können die Stabilität der Emulsion beeinträchtigen.

Alternativ können anstelle cyclischer Siloxane lineare Oligomere mit endständigen Silanolgruppen verwendet werden. Aus diesen Oligomeren wird in Gegenwart von Emulgatoren, Kondensationskatalysatoren und einer sehr kleinen Wassermenge eine Paste gebildet, in der die Polykondensation stattfindet. Anschließend wird diese Paste auf die gewünschte Konzentration verdünnt (EP 93 310 B2). Im Allgemeinen sind die Anteile an cyclischen flüchtigen Siloxanen geringer als bei der Emulsionspolymerisation von cyclischen Siloxanen. Eine Verminderung des Anteils an diesen flüchtigen Siloxanen kann z.B. dadurch erfolgen, dass erst eine Emulsion aus der Salzform des anionischen Emulgators/ Katalysators hergestellt wird, diese dann durch Zugabe von Säure aktiviert wird (EP-A 1 072 629). Das erhöht letztlich den Salzanteil in der Emulsion, was für die Stabilität nachteilig ist. Bei der Verwendung von alkoxyterminierten Siloxanoligomeren sollen ebenfalls weniger Cyclen gebildet werden (JP-A2001288269). Allerdings sind diese Oligomere aufwändiger herzustellen und damit kostenintensiver.

Spezielle Emulgatoren auf der Basis von Taurocholaten tragen ebenfalls zur Reduzierung der Menge an Cyclen bei, die bei der Emulsionskondensation von Siloxanoligomeren gebildet werden (WO 2006102010). Aber auch hier werden, wie die Ausführungsbeispiele deutlich zeigen, über 1% Octamethylcyclotetrasiloxan gebildet.

Es ist auch vorgeschlagen worden, Dimethylpolysiloxane, insbesondere Polysiloxane, die mit Trimethylsiloxygruppen terminiert sind, mit Viskositäten von bis zu 5000000 cSt zu emulgieren, indem diese mit 10-30% bezogen auf Siloxan eines Phosphorsäurepartialesters vermischt und erhitzt werden, bis eine klare Lösung entsteht, die nach Neutralisation mit Wasser verdünnt wird (DE-A 27 30 923). Allerdings hat dieses Verfahren den Nachteil, dass das Polydimethylsiloxan dabei meist depolymerisiert wird, so dass die erhaltene Emulsion ein niedrig viskoses Siloxan und einen hohen Anteil an flüchtigen cyclischen Siloxanen z.B. Octamethylcyclotetrasiloxan enthält.

In JP2002020490 wird vorgeschlagen, als Emulgatoren mindestens eine Zweierkombination von Polyoxyethylenalkylsulfaten, Polyoxyethylenalkylphosphaten und Alkylsulfonaten bzw. die entsprechenden Säuren einzusetzen, wobei bevorzugt die Säure erst in der Emulsion durch Zusatz von Mineralsäuren wie Schwefelsäure frei gesetzt werden. Eine alleinige Verwendung von Polyoxyethylenalkylphosphaten soll nur zu niedermolekularen Polyorganosiloxanen führen, da deren katalytische Aktivität zu niedrig ist. Deshalb sind Kombinationen mit Sulfaten oder Sulfonaten und eine Aktivierung von Schwefelsäure notwendig. Das führt letztlich wiederum zu mehr als 1% cyclischer Siloxanoligomere, es sei denn die Reaktionszeit ist extrem kurz, wobei jedoch keine Viskositäten > 1000000 mm²/s erreicht werden.

Auf der anderen Seite werden derartige Emulsionen praktisch oft in der Art und Weise hergestellt, dass entweder mehrere Chargen diskontinierlich hergestellt und in einen Reifetank transferiert werden oder eine kontinuierliche Kampagne über einen bestimmten Zeitraum in einen Reifetank produziert wird, wo dann nach Erreichen der gewünschten Viskosität die Reaktion durch Neutralisation abgebrochen wird. Dabei ist es unvermeidlich, dass ein nicht geringer Teil der Emulsion länger als erforderlich im Tank verweilt, wodurch der Anteil an cyclischen Oligomeren das tolerable Maß übersteigt.

Gegenstand der Erfindung sind Emulsionen von Polyorganosiloxanen enthaltend
(A) Polyorganosiloxane, die eine Viskosität größer als 10 000 mm²/s, gemessen bei 25°C, aufweisen,
(B) mindestens einen Emulgator der Formel

   (RO)ₙP(O) (OH) ₍₃₋ₙ₎ (I),

   worin
   R gleich oder verschieden sein kann und einwertige Kohlenwasserstoffreste mit 4 bis 30 Kohlenstoffatomen bedeutet
   n 1 oder 2 ist,
   und/oder dessen Salze
   und
(C) Wasser,
mit der Maßgabe, dass die Emulsionen weniger als 2 Gew.-% Octaorganylcyclotetrasiloxan (D₄), bezogen auf Komponente (A), enthalten.

Die erfindungsgemäßen Emulsionen können nach dem Fachmann bekannten Verfahren hergestellt werden.

Ein weiterer Gegenstand der Erfindung ist ein Verfahren zur Herstellung der erfindungsgemäßen Emulsionen, dadurch gekennzeichnet, dass
(a) Polyorganosiloxane enthaltend Einheiten der allgemeinen Formel

   R²ₐ(R¹O)_{b}SiO_{(4-a-b)/2} (II),

   worin
   R² gleich oder verschieden sein kann und einen einwertigen, gegebenenfalls substituierten Kohlenwasserstoffrest mit 1 bis 30 Kohlenstoffatomen oder Wasserstoffatom bedeutet,
   R¹ gleich oder verschieden sein kann und Wasserstoffatom oder einen einwertigen, gegebenenfalls substituierten Kohlenwasserstoffrest bedeutet,
   a 0, 1, 2 oder 3 ist und
   b 0, 1, 2 oder 3 ist,
   mit der Maßgabe, dass die Summe a+b kleiner oder gleich 3 ist und die Organopolysiloxane 5 bis 500 Einheiten der Formel (II) enthalten,
(b) Emulgator der Formel (I), dessen OH-Gruppen gegebenenfalls teilweise neutralisiert sein können,
(c) Wasser und
   gegebenenfalls
(d) weitere Stoffe
durch Rühren und/oder Homogenisieren vermischt werden sowie die Organopolysiloxane (a) enthaltend Einheiten der Formel (II) bei Temperaturen von 0 bis 50°C kondensieren gelassen werden bis die gewünschte Viskosität erreicht ist und gegebenenfalls anschließend der Emulgator der Formel (I) mit Basen neutralisiert wird, so dass der pH-Wert der Emulsion größer als 5 ist, und gegebenenfalls weiteres Wasser (c) und/oder weitere Stoffe (d) zugegeben werden.

Als Misch- und Homogenisierwerkzeug können alle dem Fachmann bekannten Emulgiergeräte, wie beispielsweise schnelllaufende Rührer, Dissolverscheiben, Rotor-Stator-Homogenisatoren, Ultraschallhomogenisatoren und Hochdruckhomogenisatoren verschiedenster Bauart, verwendet werden.

Das erfindungsgemäße Verfahren kann kontinuierlich, semikontinuierlich oder diskontinuierlich betrieben werden.

Eine bevorzugte Ausführungsform des erfindungsgemäßen Verfahrens ist dadurch gekennzeichnet, dass
in einem 1. Schritt
(a) 100 Gewichtsteile Polyorganosiloxane enthaltend Einheiten der allgemeinen Formel

   R²ₐ(R¹O)_{b}SiO_{(4-a-b)/2} (II),

   worin
   R² gleich oder verschieden sein kann und einen einwertigen, gegebenenfalls substituierten Kohlenwasserstoffrest mit 1 bis 30 Kohlenstoffatomen oder Wasserstoffatom bedeutet,
   R¹ gleich oder verschieden sein kann und Wasserstoffatom oder einen einwertigen, gegebenenfalls substituierten Kohlenwasserstoffrest bedeutet,
   a 0, 1, 2 oder 3 ist und
   b 0, 1, 2 oder 3 ist,
   mit der Maßgabe, dass die Summe a+b kleiner oder gleich 3 ist und die Organopolysiloxane 5 bis 500 Einheiten der Formel (II) enthalten,
(b) 1 bis 30 Gewichtsteile eines Emulgators der Formel (I), dessen OH-Gruppen gegebenenfalls teilweise neutralisiert sein können,
(c) 1 bis 50 Gewichtsteile Wasser und
   gegebenenfalls
(d) weitere Stoffe
durch Rühren und/oder Homogenisieren vermischt werden,
in einem gegebenenfalls durchgeführten 2. Schritt
weiteres Wasser (c) zugegeben wird,
in einem 3. Schritt
die Organopolysiloxane (a) enthaltend Einheiten der Formel (II) bei Temperaturen von 0 bis 50°C kondensieren gelassen werden bis die gewünschte Viskosität erreicht ist,
in einem gegebenenfalls durchgeführten 4. Schritt
der Emulgator der Formel (I) mit Basen neutralisiert wird, so dass der pH-Wert der Emulsion größer als 5 ist und
in einem gegebenenfalls durchgeführten 5. Schritt
die im 4. Schritt erhaltene Emulsion mit weiterem Wasser (c) und/oder weiteren Stoffen (d) vermischt wird.

Bei den Polyorganosiloxanen (A), welche in den erfindungsgemäßen Emulsionen enthalten sind, handelt es sich vorzugsweise um solche enthaltend Einheiten der Formel (II), besonders bevorzugt um solche aus Einheiten der Formel (II) mit einem durchschnittlichen Wert von a von 1,990 bis 2,005 und einem durchschnittlichen Wert von b von 0,001 bis 0,004, insbesondere um solche aus Einheiten der Formel (II) mit R¹ gleich Wasserstoffatom, R² gleich Methylrest und einem durchschnittlichen Wert von a von 1,990 bis 2,005 und einem durchschnittlichen Wert von b von 0,001 bis 0,004. Ganz besonders bevorzugt handelt es sich bei den Polyorganosiloxanen (A) um Dimethylpolysiloxane, die Trimethylsiloxy- und/oder Dimethylhydroxysiloxyendgruppen tragen.

Polyorganosiloxane (A), welche in den erfindungsgemäßen Emulsionen enthalten sind, haben eine Viskosität von vorzugsweise größer als 100 000 mm²/s, besonders bevorzugt größer als 1 000 000 mm²/s, jeweils bei 25°C.

Beispiele für Reste R sind verzweigte oder unverzweigte Alkylreste mit 4 bis 30 Kohlenstoffatomen, wie Butyl-, Hexyl-, 2-Ethylhexyl-, Octyl-, Isononyl-, n-Decyl-, Dodecyl-, Isotridecyl- und n-Tetradecylreste, ungesättigte aliphatische Reste, wie Oleylreste, sowie aromatische Reste, wie Phenyl-, Toloyl-, Xylyl-, Nonylphenyl-, Naphthyl-, Anthracyl-, Tristyrylphenyl- oder Benzylreste.

Bevorzugt handelt es sich bei Rest R um Alkylreste mit 4 bis 18 Kohlenstoffatomen, besonders bevorzugt um n-Butyl-, n-Octyl-, 2-Ethylhexyl-, n-Decyl-, n-Dodecyl- oder n-Tetradecylreste, insbesondere um n-Octyl- und n-Decylreste.

Beispiele für erfindungsgemäß eingesetzte Verbindungen der Formel (I) sind Di-n-butylphosphat, Di-n-hexylphosphat, Mono-n-octylphosphat, Di-n-octylphosphat, Mono-2-ethylhexylphosphat, Di-2-ethylhexylphosphat, Mono-i-nonylphosphat, Di-i-nonylphosphat, Mono-n-decylphosphat, n-Octyl-n-Decylphosphat, Di-n-decylphosphat, Monoisotridecylphosphat, Di-n-nonylphenylphosphat, Monooleylphosphat und Distearylphosphat.

Bevorzugt handelt es sich bei den erfindungsgemäß eingesetzten Verbindungen der Formel (I) um Mono-n-octylphosphat, Di-n-octylphosphat, Mono-n-decylphosphat, n-Octyl-n-Decylphosphat und Di-n-decylphosphat.

Vorzugsweise handelt es sich bei den erfindungsgemäß eingesetzten Verbindungen der Formel (I) um Mischungen von Diestern und Monoestern.

Die erfindungsgemäßen Emulsionen können als Komponente (B) nun Verbindungen der Formel (I) als solche enthalten oder deren Salze, bevorzugt mit Alkali- bzw. Erdalkalihydroxiden, Ammoniak oder Aminen, oder Gemische aus Säuren der Formel (I) und deren Salze.

Bevorzugt handelt es sich bei Komponente (B) der erfindungsgemäßen Emulsionen um Salze der Verbindungen der allgemeinen Formel (I), insbesondere um Alkalisalze oder Triethanolaminsalze.

Die Säurezahl der in der erfindungsgemäßen Emulsion enthaltenen Komponente (B) wird durch deren Anzahl an freien OH-Gruppen sowie deren Molmasse bestimmt, also die Menge an KOH in mg, die zur Neutralisation von 1 g Komponente (B) benötigt wird. Die Säurezahl der Komponente (B) liegt vorzugsweise im Bereich von 0 bis 200, besonders bevorzugt im Bereich von 0 bis 20, insbesondere bei 0, d.h. als Komponente (B) enthalten die erfindungsgemäßen Emulsionen in diesem Fall vollständig neutralisierte Verbindungen der Formel (I).

Verbindungen der Formel (I) sind kommerziell verfügbar bzw. nach allgemein bekannten chemischen Methoden darstellbar.

Die erfindungsgemäßen Emulsionen enthalten vorteilhafterweise keine bzw. einen sehr geringen Anteil an cyclischen Siloxanen, insbesondere an Octaorganylcyclotetrasiloxanen (D₄). Die Organylgruppen in den Cyclosiloxanen richten sich nach den Organylgruppen im eingesetzten Organopolysiloxan und sind bevorzugt Methylgruppen.

Die erfindungsgemäße Emulsion enthält bevorzugt weniger als 1 Gew.-%, besonders bevorzugt weniger als 0,5 Gew.-%, Octaorganylcyclotetrasiloxan, insbesondere Octamethylcyclotetrasiloxan(D₄), jeweils bezogen auf Komponente (A).

Die erfindungsgemäße Emulsion hat einen Teilchendurchmesser von vorzugsweise 50 bis 1000 nm, besonders bevorzugt von 100 bis 500 nm, insbesondere von 100 bis 200 nm, wobei sich diese Angaben auf den Mittelwert der Volumenverteilung gemessen nach dem Prinzip der Fraunhoferbeugung (entsprechend ISO 13320) beziehen.

Die erfindungsgemäßen Emulsionen haben einen Gehalt an nichtflüchtigen Anteilen gemessen nach DIN EN ISO 3251 von bevorzugt 1 bis 80 Gew.-%, besonders bevorzugt von 10 bis 65 Gew.-%, insbesondere von 30 bis 60 Gew.-%.

Der pH-Wert der erfindungsgemäßen Emulsion beträgt vorzugsweise 5 bis 10, besonders bevorzugt 6 bis 8, insbesondere etwa 7.

Beispiele für Kohlenwasserstoffreste R² sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neoPentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Octadecylreste, wie der n-Octadecylrest; Cycloalkylreste, wie der Cyclopentyl-, Cyclohexyl-, Cycloheptylrest und Methylcyclohexylreste; Alkenylreste, wie der Vinyl-, l-Propenyl- und der 2-Propenylrest; Arylreste, wie der Phenyl-, Naphthyl-, Anthryl- und Phenanthrylrest; Alkarylreste, wie o-, m-, p-Tolylreste; Xylylreste und Ethylphenylreste; und Aralkylreste, wie der Benzylrest, der α- und der β-Phenylethylrest.

Beispiele für substituierte Reste R² sind mit Halogen-, Cyano-, Glycidoxy-, Polyalkylenglycol- oder Aminogruppen substituierte Reste, wie beispielsweise Trifluorpropyl-, Cyanoethyl-, Glycidoxypropyl-, Polyalkylenglycolpropyl-, Aminopropyl- oder Aminoethylaminopropylreste.

Bevorzugt hat in den Einheiten der Formel (II) maximal 1 Rest R² die Bedeutung von Wasserstoffatom.

Bevorzugt handelt es sich bei Rest R² um Kohlenwasserstoffreste mit 1 bis 18 Kohlenstoffatomen, besonders bevorzugt um den Methyl- oder den Phenylrest, wobei insbesondere mehr als 80 Mol-% der Reste R² im Siloxan (a) die Bedeutung von Methylreste haben.

Beispiele für Reste R¹ sind die für Reste R² angegebenen Beispiele.

Bevorzugt handelt es sich bei Rest R¹ um Wasserstoffatom und Kohlenwasserstoffreste mit 1 bis 4 Kohlenstoffatomen, besonders bevorzugt um Wasserstoffatom.

In Formel (II) hat die Summe a+b einen Wert von bevorzugt durchschnittlich 1,5 bis 2,4, besonders bevorzugt durchschnittlich 1,8 bis 2,3, insbesondere durchschnittlich 1,9 bis 2,1.

Die im ersten Schritt des erfindungsgemäßen Verfahrens eingesetzten Siloxane (a) bestehen bevorzugt aus 5 bis 500, besonders bevorzugt aus 10 bis 200, insbesondere aus 20 bis 100, Einheiten der Formel (II).

In bevorzugt 0,4 bis 40 %, besonders bevorzugt 2 bis 10 %, der Einheiten der Formel (II) der im ersten Schritt des erfindungsgemäßen Verfahrens eingesetzten Siloxane (a) ist b ungleich 0.

Beispiele für erfindungsgemäß eingesetzten Siloxane (a) sind mit Alkoxy- oder Hydroxygruppen terminierte Polydiorganosiloxane, insbesondere Polydiethyl- und Polydimethylsiloxane.

Die im ersten Schritt des erfindungsgemäßen Verfahrens eingesetzten Siloxane (a) haben eine Viskosität von bevorzugt 5 bis 10 000 mm²/s, besonders bevorzugt 10 bis 500 mm²/s, insbesondere 30 bis 100 mm²/s, jeweils bei 25°C.

Bevorzugt handelt es sich bei den Siloxanen (a) um solche der Formel

HO[SiR²₂O]_{c}-H (III),

wobei R² eine der obengenannten Bedeutungen hat, insbesondere Methylrest, und c einen Wert von 5 bis 500, bevorzugt von 10 bis 200, besonders bevorzugt aus 20 bis 100, besitzt.

Die Polysiloxane (a) enthaltend Einheiten der Formel (II) sind handelsübliche Produkte bzw. können nach bekannten Verfahren hergestellt werden.

Beispiele für Komponente (b) sind die oben genannten Beispiele für die Verbindungen der Formel (I), gegebenenfalls im Gemisch mit deren Salze.

Die Säurezahl der im erfindungsgemäßen Verfahren eingesetzten Verbindung der Formel (I) wird durch den durchschnittlichen Wert von n und ihrer Molmasse bestimmt, also die Menge an KOH in mg, die zur Neutralisation von 1 g Verbindung der Formel (I) benötigt wird. Die Säurezahl der erfindungsgemäß eingesetzten Verbindung der Formel (I) liegt vorzugsweise im Bereich von 100 bis 600, besonders bevorzugt im Bereich von 200 bis 500, insbesondere im Bereich von 250 bis 450.

Komponente (b) wird im erfindungsgemäßen Verfahren in Mengen von vorzugsweise 1 bis 25 Gewichtsteilen, insbesondere 2 bis 10 Gewichtsteilen, jeweils bezogen auf 100 Gewichtsteile Polyorganosiloxan (a), eingesetzt.

Als Wasser (c) können alle Arten von Wässer eingesetzt werde, die auch bisher zur Herstellung von Dispersionen eingesetzt wurden.

Als Wasser (c) wird vorzugsweise teil- oder vollentsalztes Wasser, destilliertes oder (mehrfach) redestilliertes Wasser, Wässer für medizinische oder pharmazeutische Zwecke, wie z.B. gereinigtes Wasser (Aqua purificata gemäß Pharm. Eur.) eingesetzt.

Das erfindungsgemäß eingesetzte Wasser (c) hat vorzugsweise eine Leitfähigkeit von weniger als 50 µS/cm, besonders bevorzugt weniger als 10 µS/cm, insbesondere weniger als 1,3 µS/cm, jeweils bei 25°C und 1010 hPa.

Wasser (c) wird im ersten Schritt des erfindungsgemäßen Verfahrens in Mengen von vorzugsweise 1 bis 30 Gewichtsteilen, insbesondere 5 bis 20 Gewichtsteilen, jeweils bezogen auf 100 Gewichtsteile Polyorganosiloxan (a), eingesetzt.

Zusätzlich zu den Komponenten (a), (b) und (c) können im ersten Schritt des erfindungsgemäßen Verfahrens nun alle weiteren Stoffe (d), die üblicherweise Siliconemulsionen zugesetzt werden, eingesetzt werden, wie z.B. weitere Siloxane, die verschieden sind zu Komponente (a), Silane, insbesondere Alkoxysilane, weitere Emulgatoren, die verschieden sind zu Komponente (b), Verdicker und/oder Schutzkolloide sowie Additive, wie beispielsweise Konservierungsmittel, Desinfektionsmittel, Netzmittel, Korrosionsinhibitoren, Farbstoffe und Duftstoffe. Der Zusatz dieser Komponenten kann jedoch auch nach einem späteren Verfahrensschritt, z.B. nach dem 5. Schritt, erfolgen.

Beispiele für weitere Siloxane (d), die erfindungsgemäß eingesetzt werden können, sind solche der Formel (II) mit b gleich 0, wie z.B. trimethylsiloxyterminierte Polydimethylsiloxane. Solche Siloxane (d) werden vorteilhafterweise eingesetzt, um die nach der Kondensationsreaktion erhaltene Viskosität des Polysiloxans in der Emulsion zu steuern.

Falls weitere Siloxane (d) eingesetzt werden, handelt es sich um Mengen von bevorzugt 0,01 bis 10 Gewichtsteilen, bezogen auf 100 Gewichtsteile Komponente (a). Im erfindungsgemäßen Verfahren werden bevorzugt keine weiteren Siloxane (d) eingesetzt.

Beispiele für Silane (d), die erfindungsgemäß eingesetzt werden können, sind Methyltrimethoxysilan, Tetraethoxysilan, Vinyltriethoxysilan oder deren Hydrolyse-/Kondensationsprodukte. Solche Silane (d) werden vorteilhafterweise eingesetzt, um verzweigte oder vernetzte Siloxane z.B. solche, die nach der Trocknung der Emulsion elastische Filme bilden, zu erhalten. Diese Silane (d) können auch nach dem 3. Schritt zugesetzt werden.

Falls Silane (d) eingesetzt werden, handelt es sich um Mengen von bevorzugt 0,01 bis 10 Gewichtsteilen, bezogen auf 100 Gewichtsteile Komponente (a). Im erfindungsgemäßen Verfahren werden bevorzugt keine weiteren Silane (d) eingesetzt.

Beispiele für weitere Emulgatoren (d), die erfindungsgemäß eingesetzt werden können, sind alle bisher bekannten Emulgatoren, wie anionische oder nichtionische Emulgatoren, wie beispielsweise Alkylsulfate, ethoxylierte Alkylsulfate, Polyethylenglycolether und -ester von natürlichen und/oder synthetischen Alkoholen bzw. Carbonsäuren mit 8 bis 24 Kohlenstoffatomen und von natürlichen Glyceriden, Polyethylenglycolether von Alkylphenolen und Alkylpolyglycoside.

Im erfindungsgemäßen Verfahren werden vorzugsweise keine kationischen und keine amphoteren Emulgatoren eingesetzt.

Im erfindungsgemäßen Verfahren werden vorzugsweise keine weiteren anionischen Emulgatoren, insbesondere keine Alkyl- oder Alkylarylbenzolsulfonsäuren oder deren Salze, als Komponente (d) eingesetzt.

Bei den erfindungsgemäß gegebenenfalls eingesetzten weiteren Emulgatoren (d) handelt es sich vorzugsweise um nichtionische Emulgatoren, wie beispielsweise alle nichtionischen Emulgatoren, die auch bisher in Siliconemulsionen eingesetzt worden sind.

Besonders bevorzugt handelt es sich bei den nichtionischen Emulgatoren (d) um Polyoxyethlenglycolether oder -ester mit einem HLB-Wert von bevorzugt größer als 10, insbesondere größer als 13, wie z.B. Polyoxyethylenstearate mit 10 bis 40 Ethylenglycoleinheiten und Polyoxyethylenisotridecylether mit 4 bis 40 Ethylenglycoleinheiten.

Falls weitere Emulgatoren (d) eingesetzt werden, handelt es sich um Mengen von bevorzugt 1 bis 20 Gewichtsteilen, bezogen auf 100 Gewichtsteile Komponente (a). Im erfindungsgemäßen Verfahren werden bevorzugt weitere Emulgatoren (d) eingesetzt. Falls bei dem erfindungsgemäßen Verfahren als Komponente (d) Verdicker bzw. Schutzkolloide eingesetzt werden, handelt es sich bevorzugt um Acrylsäurecopolymere.

Falls Verdicker und/oder Schutzkolloide (d) eingesetzt werden, handelt es sich um Mengen von bevorzugt 0,01 bis 2 Gewichtsteilen, bezogen auf 100 Gewichtsteile Komponente (a). Im erfindungsgemäßen Verfahren wird bevorzugt kein Verdicker und/oder Schutzkolloid (d) eingesetzt.

Beispiele für Additive (d), die erfindungsgemäß eingesetzt werden können, sind z.B. dem Fachmann bekannte Konservierungsmittel, Farb- oder Duftstoffe, insbesondere Konservierungsmittel, wie Methylisothiazolinon, Chlormethylisothiazolinon, Benzylisothiazolinon, Phenoxyethanol, Methylparaben, Ethylparaben, Propylparaben, Butylparaben, Isobutylparaben, Alkalibenzoate, Alkalisorbate, Iodopropynylbutylcarbamat, Benzylalkohol und 2-Brom-2-nitropropan-1,3-diol.

Falls Additive (d) eingesetzt werden, handelt es sich um Mengen von bevorzugt 0,0005 bis 2 Gewichtsteilen, bezogen auf 100 Gewichtsteile Komponente (a). Im erfindungsgemäßen Verfahren werden bevorzugt Additive (d) eingesetzt.

Im ersten Schritt des erfindungsgemäßen Verfahrens können alle Komponenten durch Rühren und/oder Homogenisieren, z.B. in beliebiger Reihenfolge, miteinander vermischt werden, wobei die Umfangsgeschwindigkeit des Rührers und/oder Rotor-Stator Homogenisators bevorzugt größer als 5 m/s, besonders bevorzugt größer als 10 m/s ist, insbesondere 5 bis 50 m/s.

Verbindung der Formel (I) als Komponente (b) kann, falls erwünscht, bereits im ersten Schritt des erfindungsgemäßen Verfahrens teilweise mit Basen, wie z.B. Alkalihydroxiden oder Aminen, neutralisiert werden, was jedoch nicht bevorzugt ist.

Die Mischung gemäß erstem Schritt des erfindungsgemäßen Verfahrens hat einen pH-Wert von kleiner 6, vorzugsweise kleiner 5, besonders bevorzugt kleiner 4, insbesondere 1 bis 3.

Vorzugsweise ist die im ersten Schritt erhaltene Emulsion aus Komponenten (a), (b), (c) und gegebenenfalls (d) hochviskos und nicht fließfähig. Besonders bevorzugt ist es, wenn die Fließgrenze (entsprechend DIN 53019-1 und zitierten Normen) der im ersten Schritt erhaltenen Emulsion größer als 100 Pa, insbesondere größer als 1000 Pa ist.

Der erste Schritt des erfindungsgemäßen Verfahrens wird bei Temperaturen von vorzugsweise 5 bis 80°C, insbesondere 10 bis 50°C, und dem Druck der umgebenden Atmosphäre, also zwischen 900 und 1100 hPa, oder bei einem erhöhten Druck von bis zu 20000 hPa, insbesondere von bis zu 10000 hPa, durchgeführt.

Bevorzugt beträgt die Dauer des erfindungsgemäßen ersten Schrittes weniger als 4 Stunden, besonders bevorzugt weniger als 2 Stunden, insbesondere 5 bis 60 Minuten.

Die im ersten Schritt des erfindungsgemäßen Verfahrens erhaltene Mischung hat eine Partikelgröße (Mittelwert der Volumenverteilung) von bevorzugt kleiner als 1 µm, besonders bevorzugt 100 bis 500 nm, insbesondere 100 bis 200 nm.

Im gegebenenfalls durchgeführten zweiten Schritt des erfindungsgemäßen Verfahrens wird die im ersten Schritt erhaltene Emulsion, insbesondere wenn sie hochviskos bis standfest ist, mit Wasser unter Rühren und/oder Homogenisieren verdünnt, so dass eine fließfähige Emulsion gebildet wird, die vorzugsweise mehr als 50 Teile Wasser je 100 Teile Komponente (a) enthält.

Das Rühren bzw. Homogenisieren kann unter den gleichen Bedingungen erfolgen wie für den ersten Schritt beschrieben.

Der zweite Schritt des erfindungsgemäßen Verfahrens wird bei Temperaturen von vorzugsweise 5 bis 50°C, insbesondere 10 bis 30°C, und dem Druck der umgebenden Atmosphäre, also zwischen 900 und 1100 hPa, oder bei einem erhöhten Druck von bis zu 20000 hPa, insbesondere von bis zu 10000 hPa, durchgeführt. Der zweite Schritt kann in dem gleichen Behälter erfolgen wie der erste Verfahrensschritt.

Bevorzugt beträgt die Dauer des erfindungsgemäß gegebenenfalls durchgeführten zweiten Schrittes weniger als 4 Stunden, besonders bevorzugt weniger als 2 Stunden, insbesondere 5 bis 60 Minuten.

Beim erfindungsgemäßen Verfahren wird bevorzugt der zweite Schritt durchgeführt.

Im dritten Schritt des erfindungsgemäßen Verfahrens werden die Organopolysiloxane (a) kondensieren gelassen bis die Viskosität erreicht ist, wie sie für Siloxan (A) in der erfindungsgemäßen Emulsion gewünscht wird, also eine Viskosität von größer als 10 000 mm²/s, vorzugsweise größer als 100 000 mm²/s, besonders bevorzugt größer als 1 000 000 mm²/s, jeweils bei 25°C.

Bevorzugt beträgt die Dauer des erfindungsgemäßen dritten Schritts 1 bis 200 Stunden, besonders bevorzugt 8 bis 96 Stunden, insbesondere 12 bis 72 Stunden. Der dritte Schritt kann in dem gleichen Behälter erfolgen wie der erste und zweite Schritt. Die Emulsion kann aber auch in einen speziellen Behälter überführt werden, wo gegebenenfalls mehrere hintereinander hergestellte Ansätze für den dritten Schritt vermischt werden. Es ist jedoch auch möglich, den ersten und zweiten Schritt kontinuierlich und den dritten Schritt in einem Reifetank durchzuführen.

Der dritte Schritt des erfindungsgemäßen Verfahrens wird bei Temperaturen von vorzugsweise 2 bis 30°C, besonders bevorzugt 5 bis 20°C, und einem Druck der umgebenden Atmosphäre, also zwischen 900 und 1100 hPa, durchgeführt.

Die bei dem erfindungsgemäßen Verfahren gegebenenfalls als Kondensationsnebenprodukte anfallenden Alkohole, z.B. wenn R¹ in Formel (II) verschieden Wasserstoffatom ist, können in der Emulsion verbleiben oder auch entfernt werden, beispielsweise durch Destillation unter Vakuum oder durch Extraktion.

Beispiele für die im gegebenenfalls durchgeführten vierten Schritt des erfindungsgemäßen Verfahrens eingesetzten Basen sind Alkalihydroxyde, wie NaOH und KOH, sowie Amine, wie z.B. Monoethanolamin und Triethanolamin. Der pH-Wert kann grundsätzlich auch durch die Zugabe von Alkalisalzen schwacher Säuren, wie z.B. Natriumcitrat, Natriumsilikat, Kaliumacetat oder Kaliumphosphat, eingestellt werden.

Bevorzugt handelt es sich bei den Basen, die im vierten Schritt des erfindungsgemäßen Verfahrens eingesetzt werden können, um Alkali- bzw. Erdalkalihydroxide, Ammoniak und Amine, besonders bevorzugt um NaOH, KOH, Monoethanolamin und Triethanolamin.

Der pH-Wert der Emulsion nach der erfindungsgemäßen Neutralisation beträgt vorzugsweise 5 bis 10, besonders bevorzugt 6 bis 8, insbesondere etwa 7.

Der gegebenenfalls durchgeführte vierte Schritt des erfindungsgemäßen Verfahrens wird bei Temperaturen von vorzugsweise 5 bis 50°C, besonders bevorzugt 15 bis 30°C, und einem Druck der umgebenden Atmosphäre, also zwischen 900 und 1100 hPa, durchgeführt.

Beim erfindungsgemäßen Verfahren wird bevorzugt der vierte Schritt durchgeführt.

Die erfindungsgemäß erhaltenen Emulsionen können nun in einem gegebenenfalls durchgeführten 5. Schritt beliebig mit weiterem Wasser (c) und/oder weiteren Stoffen (d) vermischt werden.

Bevorzugt werden zusätzlich zu den Komponenten (a), (b), (c) und gegebenenfalls (d) im erfindungsgemäßen Verfahren keine weiteren Komponenten eingesetzt.

Bei den im erfindungsgemäßen Verfahren eingesetzten Komponenten kann es sich jeweils um eine Art einer solchen Komponente wie auch um ein Gemisch aus mindestens zwei Arten einer jeweiligen Komponente handeln.

Die erfindungsgemäßen bzw. erfindungsgemäß hergestellten Emulsionen haben den Vorteil, dass sie hochviskose Polydiorganosiloxane enthalten und einen niedrigen Gehalt an Cyclen aufweisen.

Des Weiteren haben die erfindungsgemäßen bzw. erfindungsgemäß hergestellten Emulsionen den Vorteil, dass sie sehr stabil und damit lange haltbar sind.

Die erfindungsgemäßen bzw. erfindungsgemäß hergestellten Emulsionen haben den Vorteil, dass sie lagerstabil sind und ausgezeichnete anwendungstechnische Eigenschaften besitzen, wie z.B. eine sehr gute Wirkung als Trenn- und Gleitmittel, eine gute Benetzungsfähigkeit auf unterschiedlichen Substraten, eine gute Konditionerwirkung in Haarpflegeprodukten, d.h. deutliche Reduzierung der Nass- und Trockenkämmkraft.

Das erfindungsgemäße Verfahren hat den Vorteil, dass auf einfache und kostengünstige Art Emulsionen mit hochmolekularen Siloxanen hergestellt werden können.

Das erfindungsgemäße Verfahren hat ferner den Vorteil, dass auch nach längerer Dauer des dritten Schritts der Anteil an cyclischen Siloxanen gering bleibt, was z.B. bei einer kontinuierlichen Produktion mit einem breiteren Verweilzeitbereich besonders günstig ist.

Das erfindungsgemäße Verfahren hat den Vorteil, dass die Viskosität des Öles in einem weiten Bereich variiert und einfach eingestellt werden kann, ohne dass ein erhöhter Anteil an cyclischen Siloxanen gebildet wird.

Die erfindungsgemäß bzw. erfindungsgemäß hergestellten Emulsionen sind für alle Zwecke einsetzbar, für die auch bisher Emulsionen mit hochviskosen Siloxanen eingesetzt worden sind, wie beispielsweise als Trennmittel, Gleitmittel, Hydrophobiermittel und zur Textilimprägnierung, bei der Verarbeitung von Gummi und Kunststoffen oder bei der Metallverarbeitung, Hydrophobiermittel für Glas und mineralische Baustoffe oder als Bestandteil von Körperpflegeprodukten.

Ein weiterer Gegenstand der Erfindung sind Körperpflegemittel enthaltend erfindungsgemäße Emulsionen in Mengen von 0,05 bis 10 Gew.-%, besonders bevorzugt 0,5 bis 5 Gew.-%.

Vorzugsweise handelt es sich bei den erfindungsgemäßen Körperpflegemitteln um Haarpflegemittel.

Diese Haarpflegemittel enthalten neben den erfindungsgemäßen bzw. erfindungsgemäß hergestellten Emulsionen bevorzugt einen oder mehrere Konditionierer ausgewählt z.B. aus natürlichen oder synthetischen Wachsen, Pflanzenölen, Mineralölen, fluorierten Ölen, Siliconölen, insbesondere Aminsiliconölen, organischen Polymeren, die nichtionisch, anionisch, kationisch oder amphoter sein können, kationischen Proteinen und kationischen Tensiden.

Weitere Inhaltsstoffe dieser Haarpflegemittel sind z.B. Wasser, Tenside, organische Säuren, Duftstoffe, Konservierer, Vitamine, Sonnenschutzmittel sowie weitere dem Fachmann bekannte Komponenten von Haarpflegemitteln.

Bei den die erfindungsgemäßen bzw. erfindungsgemäß hergestellten Emulsionen enthaltenden Haarpflegemitteln kann es sich z.B. um Shampoos, Spülungen, Cremes, Sprays handeln. Diese Pflegemitteln verbessern sowohl die Trocken- und die Nasskämmbarkeit als auch das Griffgefühl im nassen und trockenen Haar. Die Anwendung kann z.B. beim Waschen, nach dem Waschen, als Vor- oder Nachbehandlung beim Bleichen oder beim Färben mit Direkt- oder Oxidationsfarbstoffen, und bei der permanenten Verformung der Haare (z.B. Dauerwelle) erfolgen.

Ein weiterer Gegenstand der Erfindung sind Haarpflegemittel enthaltend erfindungsgemäße Emulsionen sowie mindestens einen Konditionierer.

In den nachfolgenden Beispielen beziehen sich alle Angaben von Teilen und Prozentsätzen, soweit nicht anders angegeben, auf das Gewicht. Sofern nicht anders angegeben, werden die folgenden Beispiele bei einem Druck der umgebenden Atmosphäre, also bei etwa 1010 hPa, und bei Raumtemperatur, also etwa 25°C bzw. einer Temperatur, die sich beim Zusammengeben der Reaktanten bei Raumtemperatur ohne zusätzliche Heizung oder Kühlung einstellt, durchgeführt. Alle in den Beispielen angeführten Viskositätsangaben beziehen sich auf eine Temperatur von 25°C.

Die in den nachfolgenden Beispielen hergestellten Emulsionen wurden wie folgt geprüft:
Die Teilchengröße wurde an der dynamischen Lichtstreuung mit einem Beckmann-Coulter LS 230 bestimmt. Die angegebenen Werte beziehen sich immer auf den Mittelwert der Volumenverteilung (D[4, 3]).

Zur Bestimmung der Ölviskosität wurden 20 g Emulsion mit 30 g Aceton versetzt, wobei sich die Emulsion trennte. Die Aceton/ Wasserphase wurde abgetrennt und der Vorgang noch einmal wiederholt. Anschließend wurde das Polymer dreimal mit Wasser gewaschen und bei 110°C unter Rühren getrocknet, bis keine Wassertröpfchen mehr zu sehen waren, und anschließend noch 8h bei 110°C im Trockenschrank nachbehandelt. Die Viskosität wurde mit einem Kegel-Platte-Viskosimeter MCR 300 (Paar-Physika) bei 25°C und einem Schergefälle von l/s bestimmt.

Zur Bestimmung des Gehaltes an Octamethylcyclotetrasiloxan (D₄) wurde ein ²⁹Si -NMR Spektrum der Emulsion aufgenommen (Avance 400 Fa. Bruker, 10 mm selektiver ²⁹Si NMR-Probenkopf, Zusatz von 15% D₂O zur Originalemulsion, Pulswinkel 30° Wartezeit 30 s, 400 scans).

Aus den Integralen der Signale zwischen -19,75 bis -20 ppm (D₄) und -21,5 bis -23,25 (restliche D-Einheiten) wurde der D₄-Anteil in mol% Si ermittelt, der aufgrund der gleichen Molmasse der einzelnen Siloxaneinheit (74g/mol) praktisch gleich dem Anteil an D₄ in Gew.-% bezogen auf Polydimethylsiloxane ist.

### Beispiel 1

100 Teile eines mit α,ω-hydroxyterminierten Polydimethylsiloxans mit einer Viskosität von 60 mPas werden in einem Becher vorgelegt.

Mit einem Rotor-Stator-Homogenisator (Ultra-Turrax, Umfangsgeschwindigkeit 16 m/s) werden 6 Teile eines n-Butylphosphats mit einer Säurezahl von 470 mg KOH/g (erhältlich unter dem Namen "SERVOXYL VPIZ" bei der Elementis GmbH, D-Köln), 10 Teile eines ethoxylierten Isotridecylalkohols (erhältlich unter dem Namen "Lutensol TO 109" bei der BASF SE D-Ludwigshafen) und 10 Teile Wasser zugegeben und 5 min homogenisiert. Die entstehende standfeste gelartige Phase hatte eine Fließgrenze von 1300 Pa. Diese Phase wurde homogenisiert, bis eine Teilchengröße kleiner 500 nm erreicht wurde. Anschließend wurde die Emulsion innerhalb von 10 min mit 100 Teilen Wasser verdünnt und bei 15°C gelagert. Diese Emulsion hatte einen pH-Wert von 1,3. Nach 72 h wird die Emulsion mit Triethanolamin auf einen pH-Wert von 7 eingestellt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 2

100 Teile eines mit α,ω-hydroxyterminierten Polydimethylsiloxans mit einer Viskosität von 60 mPas werden in einem Becher vorgelegt.

Mit einem Rotor-Stator-Homogenisator (Ultra-Turrax, Umfangsgeschwindigkeit 16 m/s) werden 10 Teile eines Octyl-Decylphosphats mit einer Säurezahl von 295 mg KOH/g (erhältlich unter dem Namen "Crodafos 810 A" bei der Croda GmbH D-Nettetal), 10 Teile eines ethoxylierten Isotridecylalkohols (erhältlich unter dem Namen "Lutensol TO 109" bei der BASF SE D-Ludwigshafen), 3 Teile Triethanolamin und 10 Teile Wasser zugegeben und 10 min homogenisiert. Die entstehende gelartige Phase (Fließgrenze 890 Pa) mit einer Teilchengröße von kleiner als 200 nm wird innerhalb von 10 min mit 100 Teilen Wasser verdünnt und bei 20°C gelagert. Diese Emulsion hatte einen pH-Wert von 2,8. Nach 168 h wird die Emulsion mit Triethanolamin auf einen pH-Wert von 7 eingestellt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 3

100 Teile eines mit α,ω-hydroxyterminierten Polydimethylsiloxans mit einer Viskosität von 60 mPas werden in einem Becher vorgelegt.

Mit einem Rotor-Stator-Homogenisator (Ultra-Turrax, Umfangsgeschwindigkeit 16 m/s) werden 4 Teile eines Octyl-Decylphosphats mit einer Säurezahl von 295 mg KOH/g (erhältlich unter dem Namen "Crodafos 810 A" bei der Croda GmbH D-Nettetal), 10 Teile eines ethoxylierten Isotridecylalkohols (erhältlich unter dem Namen "Lutensol TO 109" bei der BASF SE D-Ludwigshafen), 0,2 Teilen eines Verdickers bestehend aus einem modifizierten Polyacrylsäurederivat (erhältlich unter der Bezeichnung "Pemulen TR 2" bei Gattefosse Deutschland GmbH, Weil am Rhein) und 10 Teile Wasser zugegeben und 5 min homogenisiert. Die entstehende gelartige Phase (Fließgrenze 1520 Pa) mit einer Partikelgröße von kleiner als 200 nm wird innerhalb von 10 min mit 100 Teilen Wasser verdünnt und bei 15°C gelagert. Diese Emulsion hatte einen pH-Wert von 1,9. Nach 168 h wird die Emulsion mit Triethanolamin auf einen pH-Wert von 7 eingestellt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 4

100 Teile eines mit α,ω-hydroxyterminierten Polydimethylsiloxans mit einer Viskosität von 60 mPas werden in einem Becher vorgelegt.

Mit einem Rotor-Stator-Homogenisator (Ultra-Turrax, Umfangsgeschwindigkeit 16 m/s) werden 6 Teile eines Octyl-Decylphosphats mit einer Säurezahl von 295 mg KOH/g (erhältlich unter dem Namen "Crodafos 810 A" bei der Croda GmbH D-Nettetal), 10 Teile eines ethoxylierten Isotridecylalkohols (erhältlich unter dem Namen "Arlypon IT 16 109" bei der Cognis GmbH Düsseldorf) und 10 Teile Wasser zugegeben und 5 min homogenisiert. Die entstehende gelartige Phase (Fließgrenze 1120 Pa) mit einer Partikelgröße von kleiner als 200 nm wird innerhalb von 10 min mit 100 Teilen Wasser verdünnt und bei 20°C gelagert. Diese Emulsion hatte einen pH-Wert von 1,9. Nach 72 h wird die Emulsion mit Triethanolamin auf einen pH-Wert von 7 eingestellt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 5

Die in Beispiel 4 beschriebene Arbeitsweise wird wiederholt, mit der Abänderung, dass die Emulsion erst nach 168 h neutralisiert wurde.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 6

Die in Beispiel 4 beschriebene Arbeitsweise wird wiederholt, mit der Abänderung, dass nur 99 Teile eines mit α,ω-hydroxyterminierten Polydimethylsiloxans mit einer Viskosität von 60 mPas eingesetzt werden und zusätzlich 1 Teil eines mit Trimethylsiloxygruppen terminierten Polydimethylsiloxans mit einer Viskosität von 350 mm²/s.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 7

100 Teile eines mit α,ω-hydroxyterminierten Polydimethylsiloxans mit einer Viskosität von 60 mPas werden in einem Becher vorgelegt.

Mit einem Rotor-Stator-Homogenisator (Ultra-Turrax, Umfangsgeschwindigkeit 16 m/s) werden 3 Teile eines n-Butylphosphats mit einer Säurezahl von 470 mg KOH/g (erhältlich unter dem Namen "SERVOXYL VPIZ" bei der Elementis GmbH, D-Köln), 3 Teile eines 2-Ethylhexyl-Butylphosphates mit einer Säurezahl von 310 mg KOH/g (erhältlich unter dem Namen "SERVOXYL VPTZ" bei der Elementis GmbH, D-Köln), 10 Teile eines ethoxylierten Isotridecylalkohols (erhältlich unter dem Namen "Lutensol TO 109" bei der BASF SE D-Ludwigshafen) und 10 Teile Wasser zugegeben und 10 min homogenisiert. Die entstehende standfeste gelartige Phase hatte eine Fließgrenze von 2100 Pa. Diese Phase wurde homogenisiert, bis eine Teilchengröße kleiner 500 nm erreicht wurde. Anschließend wurde die Emulsion innerhalb von 15 min mit 100 Teilen Wasser verdünnt und bei 15°C gelagert. Diese Emulsion hatte einen pH-Wert von 1,5. Nach 168 h wird die Emulsion mit Triethanolamin auf einen pH-Wert von 7 eingestellt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 8

100 Teile eines mit α,ω-hydroxyterminierten Polydimethylsiloxans mit einer Viskosität von 60 mPas werden in einem Becher vorgelegt.

Mit einem Rotor-Stator-Homogenisator (Ultra-Turrax, Umfangsgeschwindigkeit 16 m/s) werden 18 Teile eines Octyl-Decylphosphats mit einer Säurezahl von 295 mg KOH/g (erhältlich unter dem Namen "Crodafos 810 A" bei der Croda GmbH D-Nettetal), 3 Teile Triethanolamin und 35 Teile Wasser zugegeben und 5 min homogenisiert. Die entstehende viskose Phase (Fließgrenze 150 Pa) mit einer Partikelgröße von kleiner als 1000 nm wird innerhalb von 5 min mit 100 Teilen Wasser verdünnt und bei 20°C gelagert. Diese Emulsion hatte einen pH-Wert von 1,7. Nach 24 h wird die Emulsion mit Triethanolamin auf einen pH-Wert von 7 eingestellt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 9

950 kg eines mit α,ω-hydroxyterminierten Polydimethylsiloxans mit einer Viskosität von 60 mPas werden in einem Mischrührwerk mit einem Volumen von 2000 1 (Becomix RW 2000) vorgelegt. Der Homogenisator wird angeschaltet und auf eine Umfangsgeschwindigkleit von 24 m/s eingestellt. 50 kg eines n-Butylphosphats mit einer Säurezahl von 470 mg KOH/g (erhältlich unter dem Namen "SERVOXYL VPIZ" bei der Elementis GmbH, D-Köln), 100 kg eines ethoxylierten Isotridecylalkohols (erhältlich unter dem Namen "Lutensol TO 109" bei der BASF SE D-Ludwigshafen) und 100 Teile Wasser werden zugegeben und 15 min homogenisiert. Es wurde eine standfeste gelartige Phase gebildet, die eine Fließgrenze von 1050 Pa hatte. Diese Phase wurde weitere 45 min homogenisiert, bis eine Teilchengröße kleiner 500 nm erreicht wurde. Anschließend wurde die Emulsion innerhalb von 10 min mit 900 Teilen Wasser verdünnt und bei 15°C gelagert. Diese Emulsion hatte einen pH-Wert von 1,3. Nach 72 h wird die Emulsion mit Triethanolamin auf einen pH-Wert von 7 eingestellt. Anschließend wurden 1,8 kg Konservierungsmittel auf der Basis von Isothiazolinonen (erhältlich unter der Bezeichnung "Kathon CG" bei Acima Chemical Industries Ltd. CH-9471 Buchs/SG) zugegeben.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 10

In einer kontinuierlichen Emulgieranlage werden parallel 1000 kg/h eines mit α,ω-hydroxyterminierten Polydimethylsiloxans mit einer Viskosität von 60 mPas in einem Becher vorgelegt. 60 kg/h eines n-Butylphosphats mit einer Säurezahl von 470 mg KOH/g (erhältlich unter dem Namen "SERVOXYL VPIZ" bei der Elementis GmbH, D-Köln), 100 kg/h eines ethoxylierten Isotridecylalkohols (erhältlich unter dem Namen "Lutensol TO 109" bei der BASF SE D-Ludwigshafen) und 100 kg/h Wasser in einen Rotor Stator-Homogenisator, der eine Umfangsgeschwindigkeit von 30 m/s aufweist, werden dosiert. Nach diesem Homogenisator werden 1000 kg/h Wasser dosiert und in einem zweiten Mischer homogenisiert. Die entstehende Emulsion wird kontinuierlich in einen 10 m³ Lagertank gepumpt und darin unter Rühren bei 15°C gelagert. Nach 60 h wird die Emulsion mit Triethanolamin auf einen pH-Wert von 7 eingestellt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 11

100 Teile eines mit α,ω-hydroxyterminierten Polydimethylsiloxans mit einer Viskosität von 60 mPas werden in einem Becher vorgelegt.

Mit einem Flügelrührer werden 18 Teile eines Octyl-Decylphosphats mit einer Säurezahl von 295 mg KOH/g (erhältlich unter dem Namen "Crodafos 810 A" bei der Croda GmbH D-Nettetal), 3 Teile Triethanolamin und 75 Teile Wasser zugegeben und 5 min verrührt. Die entstehende grobteilige Emulsion wird innerhalb von 5 min mit 100 Teilen Wasser verdünnt und anschließend mit einem Laborhochdruckhomogenisator (APV 1000 von APV Deutschland GmbH, D-Unna) bei einem Homogenisierdruck von 600 bar homogenisiert. Die feinteilige Emulsion wird 48 h bei 20°C gelagert und anschließend mit Triethanolamin auf einen pH-Wert von 7 eingestellt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Vergleichsbeipiel V1

100 Teile eines mit α,ω-hydroxyterminierten Polydimethylsiloxans mit einer Viskosität von 60 mPas werden in einem Becher vorgelegt.

Mit einem Rotor-Stator-Homogenisator (Ultra-Turrax, Umfangsgeschwindigkeit 16 m/s) werden 6 Teile Alkylbenzolsulfonsäure (erhältlich unter dem Namen "Marlonsäure AS 3" bei der SASOL AG, D-Marl) und 7 Teile Wasser zugegeben und 10 min homogenisiert. Die entstehende gelartige Phase wird innerhalb von 5 min mit 100 Teilen Wasser verdünnt und bei 20°C gelagert. Nach 6h wird die Emulsion mit Triethanolamin auf einen pH-Wert von 7 eingestellt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Vergleichsbeipiel V2

Die in Beispiel V1 beschriebene Arbeitsweise wird wiederholt, mit der Abänderung, dass die Emulsion erst nach 168 h neutralisiert wurde.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Vergleichsbeipiel V3

100 Teile eines mit α,ω-hydroxyterminierten Polydimethylsiloxans mit einer Viskosität von 60 mPas werden in einem Becher vorgelegt.

Mit einem Rotor-Stator-Homogenisator (Ultra-Turrax, Umfangsgeschwindigkeit 16 m/s) werden 3 Teile Alkylbenzolsulfonsäure (erhältlich unter dem Namen "Marlonsäure AS 3" bei der SASOL AG, D-Marl), 3 Teile Ammoniumlaurylsulfat (erhältlich unter dem Namen "Disponil ALS 40° bei der Cognis GmbH, D-Düsseldorf) und 7 Teile Wasser zugegeben und 10 min homogenisiert. Die entstehende gelartige Phase wird innerhalb von 15 min mit 100 Teilen Wasser verdünnt und bei 20°C gelagert. Nach 6 h wird die Emulsion mit Triethanolamin auf einen pH-Wert von 7 eingestellt.

Die so erhaltenen Emulsionen wurden nun hinsichtlich Teilchengröße, Ölviskosität und den Gehalt an Octamethylcyclotetrasiloxan D₄ untersucht. Die Ergebnisse finden sich in Tabelle 1.

### Vergleichsbeipiel V4

Die in Beispiel V3 beschriebene Arbeitsweise wird wiederholt, mit der Abänderung, dass die Emulsion erst nach 168 h neutralisiert wurde.

Die so erhaltenen Emulsionen wurden nun hinsichtlich Teilchengröße, Ölviskosität und den Gehalt an Octamethylcyclotetrasiloxan D₄ untersucht. Die Ergebnisse finden sich in Tabelle 1.

### Vergleichsbeipiel V5

Es wird entsprechend der Lehre von DE-A 2730923 verfahren.

100 Teile eines Polydimethylsiloxans mit einer Viskosität von 5000 mm²/s werden vorgelegt und 15 Teile eines Octyl-Decylphosphats mit einer Säurezahl von 295 mg KOH/g (erhältlich unter dem Namen "Crodafos 810 A" bei der Croda GmbH, D-Nettetal) wurden vermischt und auf 80°C erhitzt. Die klare Mischung wurde auf 40°C abgekühlt und 4 Teile Monoethanolamin zugegeben. Anschließend wurden 120 Teile Wasser eingerührt; es entstand eine trübe Lösung, die nach 24 h in eine Öl- und eine Wasserphase getrennt war. Die Ölphase hatte eine Viskosität kleiner 100 mm²/s und einen Gehalt von 3,0 mol% D₄, 0,4 mol% D₅, 0,3 mol% D₆ und 2,4 mol% Me₃Si-O- und Me₂(OH)Si-O- Endgruppen, also einer Kettenlänge von ca. 80. Das zeigt, dass nach diesem Verfahren keine stabilen Emulsionen erhältlich sind, aber das Polydiorganosiloxan depolymerisiert wird, also dass auf diese Weise keinesfalls Emulsionen mit hochviskosem Polydiorganosiloxanen herstellbar sind.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

**Tabelle 1:**

| Beispiel | Teilchengröße D[4,3] in nm | Ölviskosität in mm²/s | D₄ in Gew. % * |
|---|---|---|---|
| 1 | 143 | 724000 | 0,2 |
| 2 | 153 | 1110000 | 0,3 |
| 3 | 141 | 1040000 | 0,2 |
| 4 | 131 | 1510000 | 0, 1 |
| 5 | 131 | 1710000 | 0,2 |
| 6 | 139 | 978000 | 0, 1 |
| 7 | 141 | 1050000 | 0,3 |
| 8 | 394 | 2520000 | 0,3 |
| 9 | 138 | 1390000 | 0,2 |
| 10 | 130 | 1260000 | 0,2 |
| 11 | 344 | 512000 | 0,2 |
| V1 | 202 | 1880000 | 1,7 |
| V2 | 202 | 1970000 | 5,4 |
| V3 | 608 | 735000 | 1,1 |
| V4 | 608 | 2600000 | 4,2 |
| V5 | - | < 100 | 3,0 |

| | | | |
|---|---|---|---|
| * bezogen auf das hochmolekulare Polyorganosiloxan der jeweiligen Emulsion | | | |

### Beispiel 12

Ein Shampoo wird wie folgt formuliert (Die Komponenten werden entsprechend der INCI-Nomenklatur bezeichnet):
0,2 Teile Guar Hydroxypropyltrimoniumchlorid (erhältlich unter dem Namen N-Hance^{®} 3000 bei der Hercules Inc.) werden in 11,92 Teilen Wasser dispergiert. 71,7 Teile Sodium Laureth Sulfat (erhältlich unter dem Namen Genapol LRO 26,5% bei der Clariant GmbH) werden langsam eingerührt und die Mischung wird auf 75°C erwärmt. Dabei werden 0,3 Teile PEG-150 Distearate (erhältlich unter dem Namen Emulgin EO 33 bei der Cognis Deutschland GmbH) bei Erreichen von 50°C zugegeben, und wenn 65°C erreicht sind, 1,2 Teile Glycol Distearate (erhältlich unter dem Namen Genapol PMS bei der Clariant GmbH). Die Mischung wird gemischt, bis 75°C erreicht sind. Dann wird die Mischung abgekühlt. Wenn 35°C erreicht sind, werden 0,6 Teile Konservierer Kathon CG (erhältlich bei der Acima Chemical Industries Ltd.Inc. CH-9471 Buchs) und 4 Teile der Emulsion von Beispiel 4 zugegeben und 5 Minuten gerührt. Abschließend werden 10,06 Teile Cocamidopropyl Betaine (erhältlich unter dem Namen Genagen CAB 30% bei der Clariant GmbH) und 0,56 Teile Natriumchlorid zugegeben und jeweils 10 Minuten gerührt. Dieses Shampoo verbessert sowohl die Trocken- und die Nasskämmbarkeit als auch das Griffgefühl im nassen und trockenen Haar.

## Patentansprüche

1. Emulsionen von Polyorganosiloxanen enthaltend
(A) Polyorganosiloxane, die eine Viskosität größer als 10 000 mm²/s, gemessen bei 25°C, aufweisen,
(B) mindestens einen Emulgator der Formel
(RO)ₙP(O) (OH) ₍₃₋ₙ₎ (I),
worin
R gleich oder verschieden sein kann und einwertige Kohlenwasserstoffreste mit 4 bis 30 Kohlenstoffatomen bedeutet,
n 1 oder 2 ist,
und/oder dessen Salze
und
(C) Wasser,
mit der Maßgabe, dass die Emulsionen weniger als 2 Gew.-% Octaorganylcyclotetrasiloxan (D₄), bezogen auf Komponente (A), enthalten.

2. Emulsionen gemäß Anspruch 1, **dadurch gekennzeichnet, dass** sie weniger als 1 Gew.-% Octaorganylcyclotetrasiloxan (D₄), bezogen auf Komponente (A), enthalten.

3. Emulsionen gemäß Anspruch 1 oder 2, **dadurch gekennzeichnet, dass** Polyorganosiloxane (A) eine Viskosität größer als 100 000 mm²/s, gemessen bei 25°C, aufweisen.

4. Emulsionen gemäß einem oder mehreren der Ansprüche 1 bis 3, **dadurch gekennzeichnet, dass** sie einen Teilchendurchmesser von 50 bis 1000 nm aufweisen.

5. Emulsionen gemäß einem oder mehreren der Ansprüche 1 bis 4, **dadurch gekennzeichnet, dass** es sich bei Verbindungen der Formel (I) um Mischungen von Diestern und Monoestern handelt.

6. Verfahren zur Herstellung der Emulsionen gemäß einem oder mehreren der Ansprüche 1 bis 5, **dadurch gekennzeichnet, dass**
(a) Polyorganosiloxane enthaltend Einheiten der allgemeinen Formel
R²ₐ(R¹O)_{b}SiO_{(4-a-b)/2} (II),
worin
R² gleich oder verschieden sein kann und einen einwertigen, gegebenenfalls substituierten Kohlenwasserstoffrest mit 1 bis 30 Kohlenstoffatomen oder Wasserstoffatom bedeutet,
R¹ gleich oder verschieden sein kann und Wasserstoffatom oder einen einwertigen, gegebenenfalls substituierten Kohlenwasserstoffrest bedeutet,
a 0, 1, 2 oder 3 ist und
b 0, 1, 2 oder 3 ist,
mit der Maßgabe, dass die Summe a+b kleiner oder gleich 3 ist und die Organopolysiloxane 5 bis 500 Einheiten der Formel (II) enthalten und in 0,4 bis 40 % der Einheiten b ungleich 0 ist,
(b) Emulgator der Formel (I), dessen OH-Gruppen gegebenenfalls teilweise neutralisiert sein können,
(c) Wasser und
gegebenenfalls
(d) weitere Stoffe
durch Rühren und/oder Homogenisieren vermischt werden sowie die Organopolysiloxane (a) enthaltend Einheiten der Formel (II) bei Temperaturen von 0 bis 50°C kondensieren gelassen werden, bis die gewünschte Viskosität erreicht ist, und gegebenenfalls anschließend der Emulgator der Formel (I) mit Basen neutralisiert wird, so dass der pH-Wert der Emulsion größer als 5 ist, und gegebenenfalls weiteres Wasser (c) und/oder weitere Stoffe (d) zugegeben werden.

7. Verfahren nach Anspruch 6, **dadurch gekennzeichnet, dass** in einem 1. Schritt
(a) 100 Gewichtsteile Polyorganosiloxane enthaltend Einheiten der allgemeinen Formel
R²ₐ(R¹O)_{b}SiO_{(4-a-b)/2} (II),
worin
R² gleich oder verschieden sein kann und einen einwertigen, gegebenenfalls substituierten Kohlenwasserstoffrest mit 1 bis 30 Kohlenstoffatomen oder Wasserstoffatom bedeutet,
R¹ gleich oder verschieden sein kann und Wasserstoffatom oder einen einwertigen, gegebenenfalls substituierten Kohlenwasserstoffrest bedeutet,
a 0, 1, 2 oder 3 ist und
b 0, 1, 2 oder 3 ist,
mit der Maßgabe, dass die Summe a+b kleiner oder gleich 3 ist und die Organopolysiloxane 5 bis 500 Einheiten der Formel (II) enthalten und in 0,4 bis 40 % der Einheiten b ungleich 0 ist,
(b) 1 bis 30 Gewichtsteile eines Emulgators der Formel (I), dessen OH-Gruppen gegebenenfalls teilweise neutralisiert sein können,
(c) 1 bis 50 Gewichtsteile Wasser und
gegebenenfalls
(d) weitere Stoffe
durch Rühren und/oder Homogenisieren vermischt werden,
in einem gegebenenfalls durchgeführten 2. Schritt weiteres Wasser (c) zugegeben wird,
in einem 3. Schritt
die Organopolysiloxane (a) enthaltend Einheiten der Formel (II) bei Temperaturen von 0 bis 50°C kondensieren gelassen werden bis die gewünschte Viskosität erreicht ist,
in einem gegebenenfalls durchgeführten 4. Schritt der Emulgator der Formel (I) mit Basen neutralisiert wird, so dass der pH-Wert der Emulsion größer als 5 ist und
in einem gegebenenfalls durchgeführten 5. Schritt die im 4. Schritt erhaltene Emulsion mit weiterem Wasser (c) und/oder weiteren Stoffen (d) vermischt wird.

8. Verfahren nach Anspruch 6 oder 7, **dadurch gekennzeichnet, dass** im ersten Schritt die Umfangsgeschwindigkeit des Rührers und/oder Rotor-Stator Homogenisators größer als 5 m/s ist.

9. Körperpflegemittel enthaltend Emulsionen gemäß einem oder mehreren der Ansprüche 1 bis 5 in Mengen von 0,05 bis 10 Gew.-%.

10. Körperpflegemittel gemäß Anspruch 9, **dadurch gekennzeichnet, dass** es sich um eine Haarpflegemittel handelt und mindestens einen Konditionierer enthält.

## Claims

1. Emulsion of polyorganosiloxanes comprising
(A) polyorganosiloxanes which have a viscosity greater than 10 000 mm²/s, measured at 25°C,
(B) at least one emulsifier of the formula
(RO)ₙP(O) (OH) ₍₃₋ₙ₎ (I)
in which
R can be identical or different and is monovalent hydrocarbon radicals having 4 to 30 carbon atoms,
n is 1 or 2,
and/or salts thereof
and
(C) water,
with the proviso that the emulsion comprises less than 2% by weight of octaorganylcyclotetrasiloxane (D₄), based on component (A).

2. Emulsion according to Claim 1, **characterized in that** it comprises less than 1% by weight of octaorganylcyclotetrasiloxane (D₄), based on component (A).

3. Emulsion according to Claim 1 or 2, **characterized in that** polyorganosiloxanes (A) have a viscosity greater than 100 000 mm²/s, measured at 25°C.

4. Emulsion according to one or more of Claims 1 to 3, **characterized in that** it has a particle diameter of 50 to 1000 nm.

5. Emulsion according to one or more of Claims 1 to 4, **characterized in that** compounds of the formula (I) are mixtures of diesters and monoesters.

6. Method for producing the emulsions according to one or more of Claims 1 to 5, **characterized in that**
(a) polyorganosiloxanes comprising units of the general formula
R²ₐ(R¹O)_{b}SiO_{(4-a-b)/2} (II)
in which
R² can be identical or different and is a monovalent, optionally substituted hydrocarbon radical having 1 to 30 carbon atoms or hydrogen atom,
R¹ can be identical or different and is hydrogen atom or a monovalent, optionally substituted hydrocarbon radical,
a is 0, 1, 2 or 3 and
b is 0, 1, 2 or 3,
with the proviso that the sum a+b is less than or equal to 3 and the organopolysiloxanes contain 5 to 500 units of the formula (II) and in 0.4 to 40% of the units, b is not equal to 0,
(b) emulsifier of the formula (I), the OH groups of which can optionally be partially neutralized,
(c) water and
optionally
(d) further substances
are mixed by stirring and/or homogenization, and the organopolysiloxanes (a) comprising units of the formula (II) are left to condense at temperatures of from 0 to 50°C until the desired viscosity is reached, and optionally then the emulsifier of the formula (I) is neutralized with bases such that the pH of the emulsion is greater than 5, and optionally further water (c) and/or further substances (d) are added.

7. Method according to Claim 6, **characterized in that** in a 1st step
(a) 100 parts by weight of polyorganosiloxanes comprising units of the general formula
R²ₐ(R¹O)_{b}SiO_{(4-a-b)/2} (II)
in which
R² can be identical or different and is a monovalent, optionally substituted hydrocarbon radical having 1 to 30 carbon atoms or hydrogen atom,
R¹ can be identical or different and is hydrogen atom or a monovalent, optionally substituted hydrocarbon radical,
a is 0, 1, 2 or 3 and
b is 0, 1, 2 or 3,
with the proviso that the sum a+b is less than or equal to 3 and the organopolysiloxanes contain 5 to 500 units of the formula (II) and in 0.4 to 40% of the units, b is not equal to 0,
(b) 1 to 30 parts by weight of an emulsifier of the formula (I), the OH groups of which can be optionally partially neutralized,
(c) 1 to 50 parts by weight of water and
optionally
(d) further substances
are mixed by stirring and/or homogenization,
in an optionally carried out 2nd step
further water (c) is added,
in a 3rd step
the organopolysiloxanes (a) comprising units of the formula (II) are left to condense at temperatures of from 0 to 50°C until the desired viscosity is reached, in an optionally carried out 4th step
the emulsifier of the formula (I) is neutralized with bases such that the pH of the emulsion is greater than 5 and
in an optionally carried out 5th step
the emulsion obtained in the 4th step is mixed with further water (c) and/or further substances (d).

8. Method according to Claim 6 or 7, **characterized in that**, in the first step, the peripheral speed of the stirrer and/or rotor-stator homogenizer is greater than 5 m/s.

9. Bodycare composition comprising emulsions according to one or more of Claims 1 to 5 in amounts of from 0.05 to 10% by weight.

10. Bodycare composition according to Claim 9, **characterized in that** it is a haircare composition and comprises at least one conditioner.

## Revendications

1. Émulsions de polyorganosiloxanes contenant
(A) des polyorganosiloxanes qui présentent une viscosité supérieure à 10 000 mm²/s, mesurée à 25 °C,
(B) au moins un émulsifiant de formule
(RO)ₙP(O) (OH) ₍₃₋ₙ₎ (I)
dans laquelle
les R peuvent être identiques ou différents, et signifient des radicaux hydrocarbonés monovalents de 4 à 30 atomes de carbone,
n représente 1 ou 2,
et/ou ses sels,
et
(C) de l'eau,
à condition que les émulsions contiennent moins de 2 % en poids d'octaorganylcyclotétrasiloxane (D₄), par rapport au composant (A).

2. Émulsions selon la revendication 1, **caractérisées en ce qu'**elles contiennent moins de 1 % en poids d'octaorganylcyclotétrasiloxane (D₄), par rapport au composant (A).

3. Émulsions selon la revendication 1 ou 2, **caractérisées en ce que** les polyorganosiloxanes (A) présentent une viscosité supérieure à 100 000 mm²/s, mesurée à 25 °C.

4. Émulsions selon une ou plusieurs des revendications 1 à 3, **caractérisées en ce qu'**elles présentent un diamètre de particule de 50 à 1 000 nm.

5. Émulsions selon une ou plusieurs des revendications 1 à 4, **caractérisées en ce que** les composés de formule (I) consistent en des mélanges de diesters et de monoesters.

6. Procédé de fabrication des émulsions selon une ou plusieurs des revendications 1 à 5, **caractérisé en ce que**
(a) des polyorganosiloxanes contenant des unités de formule générale
R²ₐ(R¹O)_{b}SiO_{(4-a-b)/2} (II)
dans laquelle
les R² peuvent être identiques ou différents, et signifient un radical hydrocarboné monovalent, éventuellement substitué, de 1 à 30 atomes de carbone, ou un atome d'hydrogène,
les R¹ peuvent être identiques ou différents, et signifient un atome d'hydrogène ou un radical hydrocarboné monovalent, éventuellement substitué,
a représente 0, 1, 2 ou 3, et
b représente 0, 1, 2 ou 3,
à condition que la somme a+b soit inférieure ou égale à 3 et que les organopolysiloxanes contiennent 5 à 500 unités de formule (II), et que b soit différent de 0 dans 0,4 à 40 % des unités,
(b) un émulsifiant de formule (I), dont les groupes OH peuvent éventuellement être neutralisés en partie,
(c) de l'eau et
éventuellement
(d) des substances supplémentaires
sont mélangés par agitation et/ou homogénéisation, et les organopolysiloxanes (a) contenant des unités de formule (II) sont laissés se condenser à des températures de 0 à 50 °C jusqu'à ce que la viscosité souhaitée soit obtenue, puis l'émulsifiant de formule (I) est éventuellement neutralisé avec des bases, de sorte que le pH de l'émulsion soit supérieur à 5, et de l'eau (c) supplémentaire et/ou des substances (d) supplémentaires sont éventuellement ajoutées.

7. Procédé selon la revendication 6, **caractérisé en ce que**
lors d'une étape 1,
(a) 100 parties en poids de polyorganosiloxanes contenant des unités de formule générale
R²ₐ(R¹O)_{b}SiO_{(4-a-b)/2} (II)
dans laquelle
les R² peuvent être identiques ou différents, et signifient un radical hydrocarboné monovalent, éventuellement substitué, de 1 à 30 atomes de carbone, ou un atome d'hydrogène,
les R¹ peuvent être identiques ou différents, et signifient un atome d'hydrogène ou un radical hydrocarboné monovalent, éventuellement substitué,
a représente 0, 1, 2 ou 3, et
b représente 0, 1, 2 ou 3,
à condition que la somme a+b soit inférieure ou égale à 3 et que les organopolysiloxanes contiennent 5 à 500 unités de formule (II) et que b soit différent de 0 dans 0,4 à 40 % des unités,
(b) 1 à 30 parties en poids d'un émulsifiant de formule (I), dont les groupes OH peuvent éventuellement être neutralisés en partie,
(c) 1 à 50 parties en poids d'eau et
éventuellement
(d) des substances supplémentaires
sont mélangés par agitation et/ou homogénéisation,
lors d'une étape 2 éventuellement réalisée,
de l'eau (c) supplémentaire est ajoutée,
lors d'une étape 3,
les organopolysiloxanes (a) contenant des unités de formule (II) sont laissés se condenser à des températures de 0 à 50 °C jusqu'à ce que la viscosité souhaitée soit obtenue,
lors d'une étape 4 éventuellement réalisée, l'émulsifiant de formule (I) est neutralisé avec des bases, de sorte que le pH de l'émulsion soit supérieur à 5, et
lors d'une étape 5 éventuellement réalisée,
l'émulsion obtenue à l'étape 4 est mélangée avec de l'eau (c) supplémentaire et/ou des substances (d) supplémentaires.

8. Procédé selon la revendication 6 ou 7, **caractérisé en ce que**, dans la première étape, la vitesse périphérique de l'agitateur et/ou de l'homogénéisateur rotor-stator est supérieure à 5 m/s.

9. Agent de soin pour le corps contenant des émulsions selon une ou plusieurs des revendications 1 à 5 en quantités de 0,05 à 10 % en poids.

10. Agent de soin pour le corps selon la revendication 9, **caractérisé en ce qu'**il s'agit d'un agent de soin pour les cheveux et **en ce qu'**il contient au moins un conditionneur.
